# EUROPÄISCHE PATENTANMELDUNG

(11) **EP 2 165 701 A1**
(43) Veröffentlichungstag der Anmeldung: **24.03.2010**
(21) Anmeldenummer: 08164567.3
(22) Anmeldetag: 18.09.2008
(51) Int. Cl.: A61K 9/00, A61K 31/785, A61K 31/155

(54) **Antimikrobiell und intiviral wirkende zusammensetzung**

(71) Anmelder: Croma-Pharma Gesellschaft m.b.H., 2100 Leobendorf (AT)
(72) Erfinder: Die Erfindernennung liegt noch nicht vor
(74) Vertreter: Sonn & Partner Patentanwälte

(57) **Zusammenfassung**

Die vorliegende Erfindung betrifft eine wässrige antimikrobiell und antiviral wirkende Zusammensetzung zur Verwendung am Auge umfassend 0,5% bis 3% Hydroxypropylmethylzellulose und 0,01% bis 0,1% Polyhexanid.

## Beschreibung

Die vorliegende Erfindung betrifft eine Zusammensetzung zur Verwendung am Auge.

Mikroorganismen, besonders Bakterien können in allen Abschnitten des Auges Infektionen verursachen, die bei nicht adäquater oder zeitgerechter Therapie zu bleibenden Schäden oder schlimmstenfalls zur Erblindung eines Auges führen können. Je nachdem, wo am Auge eine Entzündung vorliegt, können unterschiedliche Symptome auftreten.

Bei der bakteriellen Bindehautentzündung (Konjunktivitis) kommt es zu einer unterschiedlich heftigen Schwellung und Rötung der Bindehaut. Die Erkrankten beklagen in der Regel ein leichtes Brennen am Auge und es kommt zu einer für diese Krankheit typischen eitrigen Absonderung. Bei anderen Formen dieser Krankheit können aber auch höckerige Veränderungen, durch Follikelbildung in der Bindehaut der Augenlider im Vordergrund stehen.

Im Gegensatz dazu ist das Leitsymptom einer bakteriellen Hornhautentzündung (Keratitis)ein unangenehmer, oftmals heftiger Schmerz, der durch die oberflächliche Verletzung der Hornhaut ausgelöst wird. Durch die dabei immer auftretenden Trübungen der Hornhaut kann das Sehvermögen in unterschiedlichem Maße eingeschränkt sein. Eine Hornhautentzündung wird häufig auch von einer Bindehautentzündung begleitet. Zur Behandlung oberflächlicher bakterieller Infektionen (Keratitis, Konjunktivitis), werden in der Literatur in der Regel lokale (topische) Antibiotika, wie Ciprofloxacin, Levofloxacin und Ofloxacin empfohlen.

Unter Uveitis versteht man eine Entzündung der Regenbogenhaut (Iris) und des Ziliarkörpers (vordere Uveitis) oder eine Entzündung der Aderhaut (hintere Uveitis). Die vordere Uveitis ist geprägt durch eine Entzündung des vorderen Augenabschnittes mit Schmerzen, Lichtempfindlichkeit, Rötung der Bindehaut und einer Trübung der vorderen Augenflüssigkeit durch Ausschwemmung gelöster Eiweißstoffe und Entzündungszellen.

Im Gegensatz zur vorderen Uveitis ist eine hintere Uveitis zumeist nicht schmerzhaft. Dort können jedoch durch die Schädigung der Netzhaut Sehstörungen auftreten. Auch die Augenhöhle kann von Infektionen betroffen sein (Orbitaphlegmone), wobei für diese eher selten auftretende Infektion des Auges zumeist Streptokokken verantwortlich sind.

Die gefährlichste bakterielle Infektion am Auge ist die sogenannte Panuveitis, bei der neben dem gesamten Auge auch Strukturen außerhalb des Auges (Augenhöhle, Augenmuskel etc.) betroffen sind, sowie die Endophthalmitis, bei der es im Rahmen einer generalisierten Blutvergiftung (Sepsis) oder durch Eindringen von Keimen während einer Augenoperation zu einer Entzündung des gesamten inneren Auges kommt.

Bei Augenoperationen ist die Infektionsprophylaxe daher von größter Bedeutung, die Augen müssen vor, während und nach jeder Operation sorgfältig mit Antiseptika behandelt werden. Besonders hohe Aufmerksamkeit hinsichtlich der Infektionsprophylaxe erfordern operative Eingriffe, bei denen das Auge eröffnet wird. Dazu gehört auch eine der wichtigsten Standardoperationen der Augenheilkunde, nämlich die Kataraktoperation, zur Behandlung der meist altersbedingten Linsentrübung (Grauer Star).

Vor allen operativen Eingriffen am Auge werden daher routinemäßig Antiseptika mit breitem Wirkungsspektrum in den Bindehautsack eingebracht. Empfohlen wird dabei derzeit die Verwendung von 5% PVP-Iod (PVP - Polyvinylpyrrolidon) oder 0.05% Chlorhexidin Lösungen die unmittelbar vor dem Eingriff für mindestens 3 min einwirken müssen (ESCRS Guidelines on Prevention, Investigation and Management of Post-Operative Endophthalmitis - Version 2).

Hansmann F et al. (Ophthalmologe (2004) 101:377-83 und Ophthalmologe (2005) 102:1043-1050) verglichen die antiseptische Wirksamkeit, Sicherheit und Verträglichkeit von Polyhexanid und PVP-Iod Lösungen bei Kataraktoperationen. Dabei wurde festgestellt, dass beide Lösungen als wirksame Oberflächenantiseptika verwendet werden können. Ein entscheidender Nachteil besteht allerdings darin, dass die antiseptische Wirkung der prä-operativ aufgetragenen Lösungen im Verlauf der Operation rasch abnimmt, da die in den Lösungen enthaltenen antiseptischen Wirkstoffe durch die permanente Spülung der Augenoberfläche laufend verdünnt werden. Ein ausreichender antimikrobieller Schutz kann durch derartige Lösungen daher nur für einige wenige Sekunden gewährleistet werden.

Es ist daher eine Aufgabe der vorliegenden Erfindung eine Zusammensetzung zur Verfügung zu stellen, die bei Operationen am Auge, insbesondere bei Kataraktoperationen, als sicheres und wirksames Antiseptikum eingesetzt werden kann, dessen keimabtötende Wirkung auf das umgebende Gewebe über den gesamten Zeitraum einer Operation anhält.

Die vorliegende Erfindung betrifft eine wässrige antiseptische Zusammensetzung zur Verwendung am Auge umfassend 0,5% bis 3% Hydroxypropylmethylzellulose und 0,01% bis 0,1% Polyhexanid.

Es hat sich überraschend gezeigt, dass eine Zusammensetzung der oben beschriebenen Art besonders gut geeignet ist, als Augenantiseptikum eingesetzt zu werden. Aufgrund der viskoelastischen Eigenschaften der erfindungsgemäßen Zusammensetzung eignet sich diese auch zur Durchführung von Augenoperationen, wie z.B. Kataraktoperationen.

Polyhexanid (Polyhexamethylenbiguanid; PHMB) ist ein weit verbreitetes Desinfektionsmittel mit antibakterieller, antimykotischer, antiviraler und antiprotozoischer Wirksamkeit, das aufgrund seiner hohen Verträglichkeit seit den 1990er Jahren auch breite therapeutische Anwendung (z.B. in der Chirurgie, Augenheilkunde und zur Wundbehandlung) findet. PHMB ist ein Polykation, dessen antibakterielle Wirksamkeit auf der Interaktion mit sauren (anionischen) Phospholipiden von Bakterienzellwänden beruht. Die neutralen Lipide von humanen Zellen werden hingegen kaum beeinflusst. PHMB weist als Antiseptikum ein sehr breites Wirkungsspektrum auf und ist z.B. auch gegen Hefen und Methicillin-resistente *Staphylococcus aureus* Stämme wirksam. Gleichzeitig ist die Gewebeverträglichkeit von PHMB als sehr gut zu bewerten.

In der Augenheilkunde wird PHMB unter anderem in Lösungen zur Kontaktlinsenaufbewahrung und zur Behandlung von Amöben-Keratitis eingesetzt.

Die Effektivität einer wässrigen Lösung von Polyhexanid für die präoperative Antiseptik bei Augenoperationen wurde in Pilotstudien belegt (siehe Hansmann et al., loc.cit.). In diesen Studien wurde das kommerziell erhältliche Antiseptikum Lavasept (Zusammensetzung pro ml: 200 mg PHMB, 10 mg Macrogol 4000, Aqua pur.) mit BSS (balanced salt solution; Zusammensetzung pro ml: 6,4 mg Natriumchlorid, 0,75 mg Kaliumchlorid, 0,48 mg Calciumchlorid-Dihydrat, 0,3 mg Magnesiumchlorid-Dihydrat, 3,9 mg Natriumacetat-Trihydrat, 1,7 mg Natriumcitrat-Bihydrat) verdünnt, so dass die Konzentration von PHMB 0,04% betrug. Vergleichbare Ergebnisse hinsichtlich der Wirksamkeit von Polyhexanid als Antiseptikum erzielten auch Koburger T et al. (GMS Krankenhaushygiene Interdisziplinär 2 (2007)).

Es hat sich überraschenderweise herausgestellt, dass Polyhexanid in der gewünschten Konzentration mit Hydroxypropylmethylzellulose ein Gel bildet, welches geeignet ist als Antiseptikum bei Augenoperationen verwendet zu werden. Zusammensetzungen mit anderen Polymeren wie z.B. Hyaluronsäure (HA)oder Natriumcarboxymethylzellulose (Na-CMC) und Polyhexanid in einem Konzentrationsbereich von 0.002% (20 ppm) bis 0.2% (2000 ppm), ergaben eine Trübung bzw. einen Niederschlag, und sind deshalb für die Anwendung bei Augenoperationen ungeeignet.

| **Polymer** | **Konzentration (% m/V)** | **PHMB Konzentration (ppm)** | **kompatibel** | **Niederschlag/ Trübung** |
|---|---|---|---|---|
| HA | 1.0 | 2000 | nein | ja |
| HA | 1.0 | 400 | nein | ja |
| HA | 1.0 | 200 | nein | ja |
| HA | 1.0 | 20 | nein | ja |
| HA | 1.0 | 2 | ja | nein |
| HA | 1.0 | - | ja | nein |
| Na-CMC | 2.0 | 2000 | nein | ja |
| Na-CMC | 2.0 | 400 | nein | ja |
| Na-CMC | 2.0 | 200 | nein | ja |
| Na-CMC | 2.0 | 20 | nein | ja |
| Na-CMC | 2.0 | 2 | ja | nein |
| Na-CMC | 2.0 | - | ja | nein |
| HPMC | 2.0 | 2000 | ja | nein |
| HPMC | 2.0 | 400 | ja | nein |
| HPMC | 2.0 | 200 | ja | nein |
| HPMC | 2.0 | 20 | ja | nein |
| HPMC | 2.0 | 2 | ja | nein |
| HPMC | 2.0 | - | ja | nein |
| HEC | 2.0 | 2000 | ja | nein |
| HEC | 2.0 | 400 | ja | nein |
| HEC | 2.0 | 200 | ja | nein |
| HEC | 2.0 | 20 | ja | nein |
| HEC | 2.0 | 2 | ja | nein |
| HEC | 2.0 | - | ja | nein |

Zwei nichtionische Derivate der Zellulose, Hydroxyethylzellulose (HEC) und Hydroxypropylmethylzellulose (HPMC), sind hingegen mit PHMB im geeigneten Konzentrationsbereich kompatibel. Allerdings weisen Formulierungen mit HEC in einer physiologisch vertretbaren Konzentration von 2% nicht die notwendige Viskosität auf und werden deshalb generell nicht für die Herstellung von Viskoelastika für die Augenchirurgie verwendet.

HPMC hat sich als besonders geeignet erwiesen, um mit Polyhexanid kombiniert zu werden. Die Hydroxypropylmethylzellulose bewirkt, dass das Antiseptikum während der Operation nicht sofort verdünnt und weggespült wird, sondern für eine ausreichend lange Zeit am Wirkort vorhanden bleibt. Dadurch ist es möglich, über einen längeren Zeitraum antiseptische Bedingungen am Auge herzustellen.

Ein besonderer Vorteil der vorliegenden Erfindung liegt auch in der höheren Verweildauer der erfindungsgemäßen Zusammensetzung an der Augenoberfläche. So ist die Verweildauer einer niedrig viskosen-dispersiven Formulierung z.B. mit 2,0% HPMC auf der Augenoberfläche rund 5 - 10 mal höher als die Verweildauer einer wässrigen physiologischen Pufferlösung (BSS)(Fennes C. (2007) Diplomarbeit Medizinische Universität Wien).

Da die antibakterielle Aktivität von PHMB auf seinen polykationischen Eigenschaften beruht, ist es von Vorteil, dass in der erfindungsgemäßen Zusammensetzung kaum ionische Inhaltsstoffe enthalten sind, da Wechselwirkungen mit ionischen Inhaltsstoffen (wie z.B. NaCl) eine Verminderung der antibakteriellen Wirkung von PHMB bewirken können.

Es hat sich erfindungsgemäß herausgestellt, dass HPMC eine bestimmte Konzentration in der erfindungsgemäßen Zusammensetzung aufweisen muss, um beispielsweise bei Kataraktoperationen wirksam eingesetzt werden zu können.

Bei einer Messung bei konstanter Scherrate 5 1/s weisen Formulierungen mit 2% HPMC eine Viskosität von 2 - 8 Pas auf, was dem bevorzugten Viskositätsbereich entspricht; die Viskosität einer Formulierung mit 2% HEC (pharmaceutical grade) liegt mit 0.4 Pas deutlich darunter und ist für eine erfindungsgemäße Anwendung nicht ausreichend.

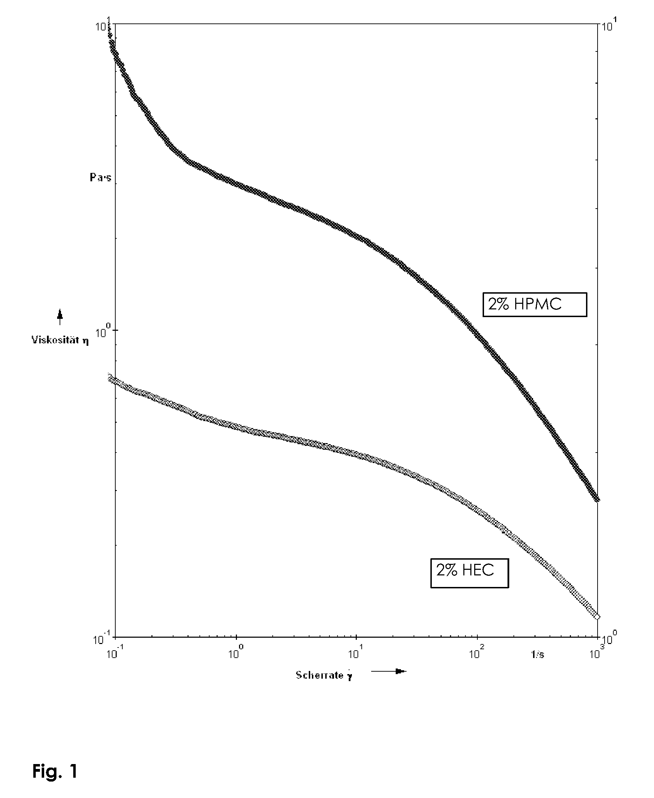

Die niedrigere Viskosität der 2% HEC Formulierung wird auch beim Vergleich der Fließkurve mit der einer 2% HPMC Lösung deutlich (siehe Fig. 1).

Daher weist die erfindungsgemäße Zusammensetzung 0,5% bis 3%, vorzugsweise 0,8% bis 2,5%, noch mehr bevorzugt 1% bis 2%, Hydroxypropylmethylzellulose auf. Liegt die Konzentration der Hydroxypropylmethylzellulose in der Zusammensetzung unter dem angegebenen Wert, weist die Zusammensetzung eine zu geringe Viskosität auf, um für den erfindungsgemäßen Zweck eingesetzt zu werden. Ist hingegen der Anteil an Hydroxypropylmethylzellulose in der Zusammensetzung höher als oben angegeben, weist die Zusammensetzung eine zu hohe Viskosität auf.

Gemäß einer bevorzugten Ausführungsform umfasst die Zusammensetzung 0,02% bis 0,08%, vorzugsweise 0,03% bis 0,06%, noch mehr bevorzugt 0,04%, Polyhexanid.

Die erfindungsgemäß verwendete Hydroxypropylmethylzellulose weist vorzugsweise ein Molekulargewicht von 5.000 bis 150.000 Da, noch mehr bevorzugt von 20.000 bis 125.000 Da, am meisten bevorzugt von 50.000 bis 110.000 Da, auf.

Ein weiterer Aspekt der vorliegenden Erfindung betrifft die Verwendung einer erfindungsgemäßen Zusammensetzung als Konjunktivalantiseptikum, als Bindehautantiseptikum oder als intrakamerales oder intravitreales Antiseptikum.

Ein noch weiterer Aspekt der vorliegenden Erfindung betrifft die Verwendung von Hydroxypropylmethylzellulose und von Polyhexanid zur Herstellung einer Zusammensetzung umfassend 0,5% bis 3% Hydroxypropylmethylzellulose und 0,01% bis 0,1% Polyhexanid zur Behandlung von Entzündungen und/oder Infektionen des Auges oder zur Infektionsprophylaxe vor und nach einer Operation am Auge.

Gemäß einer bevorzugten Ausführungsform ist die Operation am Auge eine Kataraktoperation.

Die erfindungsgemäße Zusammensetzung kann als antimikrobielles und antivirales Viskoelastikum bei der Kataraktchirurgie verwendet werden, und im Vergleich zu herkömmlichen Viskoelastika (OVDs) das Risiko einer postoperativen bakteriellen Infektion signifikant verringern.

Gleichzeitig kann das antimikrobielle und antivirale Viskoelastikum lokal auf der Augenoberfläche verwendet werden, um die Hornhaut vor und/oder nach der Operation zu befeuchten und zu desinfizieren. Es konnte gezeigt werden, dass viskoelastische Formulierungen, die eine Viskosität in einem bestimmten Bereich aufweisen, bei lokaler Applikation eine gesteigerte Verweildauer im Vergleich zu einer physiologischen Pufferlösung aufweisen. Das gilt v. a. dann, wenn das Viskoelastikum niedrig viskose-dispersive Eigenschaften aufweist, d.h. eine besonders gute Gewebebenetzung gewährleistet. Wegen der raschen und gleichmäßigen Verteilung auf der Augenoberfläche wird die optische Klarheit der Cornea nach der Applikation sehr rasch wieder hergestellt, während die hydratisierende, protektive und antiseptische Wirkung des Viskoelastikums auf die Cornea für einen längeren Zeitraum anhält.

Die vorliegende Erfindung wird anhand der Figur und den nachfolgenden Beispielen näher erläutert ohne jedoch auf diese beschränkt zu sein.

Es zeigt Fig. 1 die Fließkurve einer 2% HEC-Formulierung im Vergleich mit einer 2% HPMC-Lösung.

### Beispiele:

### Beispiel 1: Formulierung

Die getestete Formulierung enthielt als antimikrobiellen Wirkstoff PHMB (Polyhexanid, Polyhexamethylenbiguanid Hydrochlorid, CAS Nr. 32289-58-0) in einer Konzentration von 0,04%. Um eine ausreichende Viskosität, die für die Anwendung in der Kataraktchirurgie erforderlich ist, zu erreichen, wird als viskositätserhöhender Hilfsstoff HPMC (Hydroxypropylmethylzellulose, MW ∼ 86 000 Da), ein nicht-ionisches, wasserlösliches Derivat der Zellulose, in einer Konzentration von 2 % eingesetzt. Ein Beispiel einer Fließkurve ist in Fig. 1 abgebildet. Die Viskosität bei Scherrate 5 1/s liegt im Bereich von 2 bis 8 Pa.s, um eine gute Verteilung des OVDs am und im Auge zu gewährleisten.

Andere Polymere wie z.B. Hyaluronsäure (HA), Hydroxyethylzellulose (HEC) und Na-Carboxymethylcellulose (Na-CMC) wurden ebenfalls für die Herstellung einer Formulierung mit den gewünschten rheologischen Eigenschaften verwendet. Dazu wurde Hyaluronsäure in einer Konzentration von 1,0% und Na-Carboxymethylzellulose in einer Konzentration von 2,0% eingesetzt. Diese beiden Polymere waren für die Formulierungsherstellung allerdings nicht geeignet. Die Formulierungen, die PHMB enthielten, waren durch einen weißen Niederschlag getrübt (siehe Tabelle).

| **Polymer** | **Konzentration (% m/V)** | **PHMB Konzentration (ppm)** | **kompatibel** | **Niederschlag/ Trübung** |
|---|---|---|---|---|
| HA | 1.0 | 2000 | nein | ja |
| HA | 1.0 | 400 | nein | ja |
| HA | 1.0 | 200 | nein | ja |
| HA | 1.0 | 20 | nein | ja |
| HA | 1.0 | 2 | ja | nein |
| HA | 1.0 | - | ja | nein |
| Na-CMC | 2.0 | 2000 | nein | ja |
| Na-CMC | 2.0 | 400 | nein | ja |
| Na-CMC | 2.0 | 200 | nein | ja |
| Na-CMC | 2.0 | 20 | nein | ja |
| Na-CMC | 2.0 | 2 | ja | nein |
| Na-CMC | 2.0 | - | ja | nein |
| HPMC | 2.0 | 2000 | ja | nein |
| HPMC | 2.0 | 400 | ja | nein |
| HPMC | 2.0 | 200 | ja | nein |
| HPMC | 2.0 | 20 | ja | nein |
| HPMC | 2.0 | 2 | ja | nein |
| HPMC | 2.0 | - | ja | nein |
| HEC | 2.0 | 2000 | ja | nein |
| HEC | 2.0 | 400 | ja | nein |
| HEC | 2.0 | 200 | ja | nein |
| HEC | 2.0 | 20 | ja | nein |
| HEC | 2.0 | 2 | ja | nein |
| HEC | 2.0 | - | ja | nein |

Zur Einstellung eines physiologisch verträglichen pH-Wertes (für Viskoelastika zur Anwendung im Auge ist ein pH-Wert im Bereich von 6,8-7,6 erforderlich) wird zum Beispiel Phosphatpuffer eingesetzt. Die Einstellung der Osmolarität erfolgte mit Glycerin, ist aber auch mit einem anderen nicht-ionischen Polyalkohol, wie z.B. Mannit oder Sorbit, möglich.

Bei einer Messung bei konstanter Scherrate 5 1/s wiesen Formulierungen mit 2% HPMC eine Viskosität von 2 - 8 Pas auf; die Viskosität einer Formulierung mit 2% HEC (pharmaceutical grade) lag mit 0.4 Pas deutlich darunter und ist für eine erfindungsgemäße Anwendung als OVD nicht ausreichend.

Die niedrigere Viskosität der 2% HEC Formulierung wird auch beim Vergleich der Fließkurve mit der einer 2% HPMC Lösung deutlich (siehe Fig. 1) .

### Beispiel 2: Antimikrobielle Wirksamkeit

Die Bestimmung der antibakteriellen und fungiziden Wirkung von Formulierungen, die 0,04% PHMB und HPMC in unterschiedlichen Konzentrationen enthalten, wurde mittels eines quantitativen Suspensionstests nach EN1276:1997 und ISO EN1650:1997 durchgeführt (siehe Tabelle).

| **Testkeim** | **Einwirkzeit** | **Keimreduktion (log)** | | |
|---|---|---|---|---|
| | | Formulierung ohne HPMC | Formulierung mit 0,3% HPMC | Formulierung mit 2% HPMC |
| *Staphylococcus aureus* | 1 min | - | - | tbd |
| | 5 min | 6,07 | 6,07 | 6,07 |
| | 15 min | 6,07 | 6,07 | 6,07 |
| *Staphylococcus epidermidis* | 1 min | - | - | 6,19 |
| | 5 min | 6,48 | 6,48 | 6,48 |
| | 15 min | 6,48 | 6,48 | 6,48 |
| *Pseudomonas aeruginosa* | 1 min | - | - | 6,88 |
| | 5 min | 6,96 | 6,00 | 6,17 |
| | 15 min | 6,26 | 5,52 | 5,25 |
| *Candida albicans* | 1 min | - | - | 6,27 |
| | 5 min | 6,38 | 6,38 | 6,38 |
| | 15 min | 6,38 | 6,38 | 6,38 |
| *Enterococcus faecium* | 1 min | - | - | 3,89 |
| | 5 min | - | - | 6,21 |
| *Escherichia coli* | 1 min | - | - | 6,22 |
| | 5 min | - | - | 6,16 |

Mit Ausnahme von *Enterococcus faecium* wurde für alle in der Tabelle aufgelisteten Keime die geforderte Keimzahlreduktion um mindestens 5 log Einheiten innerhalb einer Minute erreicht. Bei *E. faecium,* der bekanntlich eine erhöhte Resistenz gegenüber Hitze und chemischer Inaktivierung aufweist, war eine Einwirkzeit von 5 min ausreichend, um den Keim zu inaktivieren. Die Zugabe des viskositätserhöhenden Polymers HPMC hat keinen negativen Einfluss auf die antimikrobielle Wirkung von PHMB.

## Patentansprüche

1. Wässrige antimikrobiell und antiviral wirkende Zusammensetzung zur Verwendung am und im Auge umfassend 0,5% bis 3% Hydroxypropylmethylzellulose und 0,01% bis 0,1% Polyhexanid.

2. Zusammensetzung nach Anspruch 1, **dadurch gekennzeichnet, dass** diese 0,8% bis 2,5%, vorzugsweise 1% bis 2% Hydroxypropylmethylzellulose umfasst.

3. Zusammensetzung nach Anspruch 1 oder 2, **dadurch gekennzeichnet, dass** diese 0,02% bis 0,08%, vorzugsweise 0,03% bis 0,06%, noch mehr bevorzugt 0,04%, Polyhexanid umfasst.

4. Verwendung einer Zusammensetzung nach einem der Ansprüche 1 bis 3 als Bindehautantiseptikum, Augenlidantiseptikum, Kornealantiseptikum oder intrakamerales Antiseptikum.

5. Verwendung von Hydroxypropylmethylzellulose und von Polyhexanid zur Herstellung einer Zusammensetzung umfassend 0,5% bis 3% Hydroxypropylmethylzellulose und 0,01% bis 0,1% Polyhexanid zur Prävention von Infektionen des Auges vor, während und nach einer Operation.

6. Verwendung von Hydroxypropylmethylzellulose und von Polyhexanid zur Herstellung einer Zusammensetzung umfassend 0,5% bis 3% Hydroxypropylmethylzellulose und 0,01% bis 0,1% Polyhexanid zur Behandlung einer Infektion des Auges vor und nach einer Operation.

7. Verwendung nach Anspruch 5 oder 6, **dadurch gekennzeichnet, dass** die Operation am Auge eine Kataraktoperation ist.
